# EUROPÄISCHE PATENTSCHRIFT

(11) **EP 1 252 512 B1**
(45) Veröffentlichungstag und Bekanntmachung des Hinweises auf die Patenterteilung: **09.07.2003**
(21) Anmeldenummer: 00909097.8
(22) Anmeldetag: 24.01.2000
(51) Int. Cl.: G01N 33/28

(54) **VORRICHTUNG UND VERFAHREN ZUR ERFASSUNG VON DURCH SCHMIERMITTEL BEDINGTEN BELAGBILDUNGEN AUF SENSOROBERFLÄCHEN**
DEVICE AND METHOD FOR DETECTING DEPOSIT FORMATIONS ON SENSOR SURFACES, SAID FORMATIONS BEING CAUSED BY LUBRICANTS
DISPOSITIF ET PROCEDE POUR DETECTER SUR DES SURFACES DE CAPTEUR D'UN DEPOT FORME PAR UN LUBRIFIANT

(43) Veröffentlichungstag der Anmeldung: 30.10.2002
(73) Patentinhaber: FRAUNHOFER-GESELLSCHAFT ZUR FÖRDERUNG DER ANGEWANDTEN FORSCHUNG E.V., 80636 München (DE); Bedia Motorentechnik GmbH, 91227 Leinburg (DE)
(72) Erfinder: LEIDL, Anton, D-81739 München (DE); LANGER, Peter, D-80992 München (DE); VIRNICH, Ulrich, D-92348 Berg (DE)
(74) Vertreter: Schoppe, Fritz, Dipl.-Ing.
(86) Internationale Anmeldenummer: EP0000524
(87) Internationale Veröffentlichungsnummer: WO01055718

(56) Entgegenhaltungen:
- EP-A- 0 463 796
- WO-A-96/28742
- WO-A-98/37412

## Beschreibung

Die vorliegende Erfindung bezieht sich auf eine Vorrichtung und ein Verfahren zur Erfassung von durch Schmiermittel bedingten Belagbildungen auf Sensoroberflächen. Insbesondere bezieht sich die vorliegende Erfindung auf eine solche Vorrichtung und ein solches Verfahren, die geeignet sind, um bei der Ermittlung des Alterungszustands von Schmiermitteln, insbesondere Schmierölen, die in Verbrennungsmotoren verwendet sind, eingesetzt zu werden. Darüberhinaus bezieht sich die vorliegende Erfindung insbesondere auf eine solche Vorrichtung und ein solches Verfahren, die geeignet sind, um auf Sensoroberflächen gebildete Beläge zu beseitigen. Somit eignet sich die vorliegende Erfindung insbesondere für eine "in situ"-Erfassung des Alterungszustands von Schmierölen, so daß, basierend auf einer solchen Erfassung, Ölwechselintervalle festgelegt werden können.

Schmieröle, besonders Schmieröle in Verbrennungsmotoren, werden in der Regel nach festen Zeitintervallen gewechselt. Bislang fehlen zuverlässige Sensorsysteme, die den Zustand, d.h. den Alterungszustand, eines Schmieröls zuverlässig bestimmen können, während sich das Schmieröl in dem Motor befindet. Es ist eine Anzahl unterschiedlicher Sensorsysteme bekannt, die verschiedene Parameter erfassen, um auf der Grundlage der erfaßten Parameter den Alterungszustand des Öls zu bestimmen.

Beispielsweise ist aus der DE 4131969 C2 eine Schmierölüberwachungseinrichtung bekannt, bei der die Parameter Druck, Temperatur und Viskosität sowie optional der pH-Wert, in situ im Motor erfaßt werden. Aus den erfaßten Parametern wird ein Ist-Zustand des Motoröls bestimmt. Dieser Ist-Zustand wird mit einem Soll-Zustand verglichen, wobei, wenn der Soll-Zustand hinsichtlich des Ist-Zustands eine bestimmte Abweichung zeigt, ein Alarmsignal, das anzeigt, daß ein Ölwechsel erforderlich ist, ausgegeben wird. Gemäß der DE 4131969 C2 wird der Druck mittels einer Drucksensormembran, die mit piezoresistiven Widerständen versehen ist, gemessen, die Temperatur wird mittels Platinwiderständen gemessen, der pH-Wert wird mittels eines MOS-Transistors mit einer H⁺-sensitiven Gate-Elektrode gemessen, während die Viskosität aus der Dielektrizitätszahl, die mittels Kapazitätsstrukturen erfaßt wird, oder alternativ aus der Dämpfung von Schallwellen, die mittels Piezoschwingern erzeugt werden, gemessen wird.

Aus der WO 98/05953 ist ein Oberflächenwellen-Flüssigkeitssensor bekannt, der einen Sendewandler und einen Empfangswandler aufweist, die durch jeweilige Interdigitalwandler gebildet sind. Die Anzahl von Fingerpaaren der Interdigitalwandler sowie das Material, aus dem die Elektroden bestehen, sind derart gewählt, daß die von dem Sendewandler erzeugte Volumenscherwelle und die von dem Sendewandler erzeugte Oberflächenscherwelle unterschiedliche Frequenzen aufweisen.

Darüber hinaus ist aus der WO 98/37412 eine Sensoreinrichtung zur Erfassung von Materialparametern eines flüssigen Mediums bekannt, bei der ein Oberflächenwellen-Flüssigkeitssensor zur Erfassung der Viskosität eines angrenzenden flüssigen Mediums verwendet wird, während einer der Interdigitalwandler des Oberflächenwellen-Flüssigkeitssensors ferner verwendet wird, um die Dielektrizitätszahl des anliegenden flüssigen Mediums zu bestimmen. Ferner lehrt diese Schrift als weiteren Parameter die Leitfähigkeit über die komplexe elektrische Impedanz des Interdigitalkondensators zu erfassen. Basierend auf den somit erfaßten Parametern wird, unter Berücksichtigung der Temperaturabhängigkeit, ein Ist-Zustand des flüssigen Mediums bestimmt, der mit einem Soll-Zustand verglichen wird, um somit einen Alterungszustand des flüssigen Mediums, insbesondere des Schmieröls, feststellen zu können.

Die oben beschriebenen bekannten Ölzustandssensoren ermöglichen somit die Erfassung von Viskosität, Dielektrizitätszahl und Leitfähigkeit von Ölen in situ im Motor. Dabei ermöglicht die Erfassung der Viskosität eine zuverlässigere Erfassung des Zustands der Öle als es mit einer reinen dielektrischen Messung möglich ist. Jedoch ist die Viskosität eine Meßgröße, die praktisch derzeit hauptsächlich in Laboranalysen von Motorölen bestimmt und ausgewertet wird. Bei derartigen Laboranalysen werden neben der Viskosität unter anderem auch der Feststoffgehalt, mittels einer Infrarotspektroskopie, und die Versäuerung des Öls, durch die Bestimmung der TAN (TAN = total acid number), bestimmt. Aus diesen Parametern wird dann im Labor der Ölzustand beurteilt.

Im Motorenprüfstand, d.h. bei Messungen des Ölzustands, während sich dasselbe im Motor befindet, hat sich bei der Verwendung von Oberflächenwellensensoren, wie sie oben beschrieben sind, gezeigt, daß dieselben, auch wenn sie die Erfassung der Viskosität in situ ermöglichen, unzuverlässige Ergebnisse ergaben. Darüber hinaus ist festzustellen, daß die in Laboranalysen durchführbaren Verfahren zur zuverlässigen Feststellung des Ölzustands bei einer Untersuchung des Öls im Motor aufgrund des Aufwands derselben nicht verwendet werden können.

Die Aufgabe der vorliegenden Erfindung besteht darin, eine einfache Vorrichtung und ein einfaches Verfahren zu schaffen, auf deren Grundlage eine zuverlässige Bestimmung des Alterungszustands von Schmierölen möglich ist.

Diese Aufgabe wird durch eine Vorrichtung gemäß Patentanspruch 1 und ein Verfahren gemäß Patentanspruch 7 gelöst.

Die vorliegende Erfindung schafft eine Vorrichtung zur Bestimmung, ob ein zu untersuchendes Schmiermittel eine Belagbildung auf einem Sensor bewirkt, die ein Sensorelement zum Erfassen von Werten zumindest eines physikalischen Parameters des Schmiermittels aufweist, der in Berührung mit dem Schmiermittel angeordnet ist. Ferner ist eine Einrichtung zum Anlegen einer Potentialdifferenz zwischen Sensorelement und Schmiermittel vorgesehen. Schließlich umfaßt die erfindungsgemäße Vorrichtung eine Einrichtung zur Auswertung von zumindest zwei Werten des physikalischen Parameters, die bei unterschiedlichen Potentialzuständen zwischen Sensorelement und Schmiermittel erfaßt werden, um zu bestimmen, ob eine Belagbildung auf der Sensoroberfläche durch das Schmiermittel bewirkt wird.

Die vorliegende Erfindung basiert auf der Erkenntnis, daß es bei Verwendung von Sensorelementen zur Erfassung von physikalischen Parametern von Schmiermitteln zu einer Belagbildung auf dem Sensorelement, insbesondere der Sensoroberfläche desselben, die in Berührung mit dem Schmiermittel ist, kommen kann. Eine solche Belagbildung wurde bei Messungen im Motorenprüfstand mittels eines Oberflächenwellensensors festgestellt, jedoch nicht bei Messungen im Labor, die mit denselben Schmiermittelproben, d.h. Ölproben, durchgeführt wurden. Es wurde nun herausgefunden, daß diese Belagbildung durch eine Potentialdifferenz zwischen Motorblock/Motoröl und Sensorelement hervorgerufen wird. Diese Belagbildung auf der dem Schmiermittel bzw. Öl ausgesetzten Oberfläche des Sensors beeinträchtigt jedoch die Messung der physikalischen Parameter des Öls, beispielsweise der Viskosität und dielektrischen Zahl desselben, stark.

Eine solche Belagbildung auf der Sensoroberfläche kann nun durch eine zwischen Motorblock/Motoröl und Sensor inhärent vorliegende Potentialdifferenz hervorgerufen werden. Die Belagbildung stört jedoch die Erfassung eines physikalischen Parameters des Öls durch einen entsprechenden Sensor, da durch einen solchen Belag die Ausgangssignale des Sensors verfälscht werden. Es ist nun möglich, durch das Anlegen eines elektrischen Potentials einer definierten Polarität zwischen Sensor und Schmiermittel für eine kurze Zeit, im Bereich weniger Minuten, eine weitere Belagbildung zu bewirken. Durch das Anlegen eines Potentials umgekehrter Polarität kann erreicht werden, daß der Belag wieder entfernt wird. Hierbei ist anzumerken, daß herausgefunden wurde, daß diese Belagbildung und Belagentfernung lediglich bei gealterten Schmiermitteln auftritt, während bei neuen Schmiermitteln keine Belagbildung stattfindet. Dies ist darin begründet, daß der Belag durch Inhaltsstoffe, insbesondere Feststoffe wie Ruß und dergleichen, bedingt wird, die mit zunehmender Alterung des Schmiermittels zunehmend in demselben vorliegen und eine elektrische Ladung aufweisen, so daß durch das Anlegen einer vorbestimmten Polarität zwischen Schmiermittel und Sensorelement die Belagbildung erreicht wird, während durch das Anlegen einer entgegengesetzten Polarität der Abbau eines auf dem Sensorelement vorliegenden Belags bewirkt wird.

Die vorliegende Erfindung nutzt den oben beschriebenen Effekt nun aus, um zu bestimmen, ob eine Belagbildung durch ein gealtertes Schmiermittel auf einem Sensorelement bewirkt wird, indem zumindest zwei Werte eines physikalischen Parameters des Schmiermittels mittels eines Sensorelements bei Vorliegen zweier unterschiedlicher Potentialzustände zwischen Sensorelement und Schmiermittel erfaßt werden. Basierend auf der Bestimmung kann dann die Alterung des Schmiermittels direkt ermittelt werden, da, wie oben erläutert wurde, die Belagbildung vom Alterungszustand des zu untersuchenden Schmiermittels bzw. Öls abhängt. Andererseits kann gemäß der vorliegenden Erfindung der oben genannte Effekt ausgenutzt werden, um einen auf einem Sensorelement, d.h. der in Berührung mit dem Schmiermittel befindlichen Sensoroberfläche desselben, gebildeten Belag zu beseitigen und dann eine unverfälschte Erfassung von physikalischen Parametern des Schmiermittels, beispielsweise der Viskosität, der Dielektrizitätszahl oder der Leitfähigkeit desselben, durchzuführen.

In anderen Worten ist es erfindungsgemäß durch gezieltes Anlegen einer Potentialdifferenz zwischen Sensor und Schmiermittel bzw. Öl möglich, mit einem Sensor, der auf Änderungen der physikalischen Randbedingungen an der Grenzfläche Sensor-Schmiermittel reagiert, einen Summenparameter für den Feststoffgehalt des Schmiermittels zu messen. Der durch das Anlegen der Potentialdifferenz bewirkte Belag kann durch das Anlegen einer Potentialdifferenz entgegengesetzter Polarität wieder entfernt werden. Nachdem der Sensor beschlagfrei gemacht wurde, können bei einem Sensor, der Viskositäten und/oder dielektrische Größen messen kann, mit dem gleichen Sensor, mit dem die Belagbildung bestimmt wird, die Viskosität und/oder die dielektrischen Öleigenschaften bestimmt werden.

Somit ermöglicht die vorliegende Erfindung eine zuverlässige Ölzustandsbestimmung.

Neben den Parametern, die bisher zur Beurteilung des Alterungszustands eines Öls in situ in einem Motor erfaßt wurden, beispielsweise Dielektrizitätszahl, Leitfähigkeit, Viskosität und dergleichen, kann mittels der vorliegenden Erfindung ein weiterer Summenparameter bestimmt werden, der eine Aussage über den Alterungszustand des Öls ermöglicht, nämlich, wie oben erläutert, der Parameter, ob das Schmieröl eine Belagbildung auf einem Sensorelement bewirkt. Diese Belagbildung kann von unterschiedlichen Faktoren, die mit zunehmender Alterung des Schmieröls auftreten, abhängen, insbesondere vom Eintrag von Ruß und anderen elektrisch geladenen Feststoffen in das Schmiermittel bzw. Öl. Andere Parameter, beispielsweise die Dielektrizitätszahl und die Leitfähigkeit werden dagegen insbesondere auch durch andere Veränderungen des Schmiermittels mit zunehmender Alterung, beispielsweise den Eintrag von flüssigen Stoffen, wie Wasser oder Treibstoff, sowie chemischen Verbindungen, die eine thermische Oxidation bzw. eine Versäuerung bewirken können, beeinflußt. Je mehr dieser Veränderungen des Schmiermittels erfaßt werden können, desto zuverlässiger kann der Alterungszustand des Schmiermittels beurteilt werden.

Die vorliegende Erfindung ermöglicht neben dem Erfassen eines zusätzlichen Summenparameters, nämlich der Belagbildung auf einer Sensoroberfläche durch ein Schmiermittel, zum anderen das gezielte Bilden bzw. Entfernen eines Belags, so daß physikalische Parameter des Schmiermittels, wie z.B. die Dielektrizitätszahl, die Leitfähigkeit oder die Viskosität, in einem Zustand erfaßbar sind, in dem der Sensor beschlagfrei ist. Somit ermöglicht die vorliegende Erfindung neben der Erfassung eines zusätzlichen Summenparameters auch eine exaktere Erfassung der oben genannten physikalischen Parameter.

Bevorzugte Ausführungsbeispiele der vorliegenden Erfindung werden nachfolgend bezugnehmend auf die beiliegenden Zeichnungen näher erläutert. Es zeigen:
- Fig. 1: eine schematische Darstellung einer erfindungsgemäßen Vorrichtung zur Bestimmung, ob ein zu untersuchendes Schmiermittel eine Belagbildung auf einem Sensorelement bewirkt;
- Fig. 2: eine schematische Darstellung eines bevorzugten Ausführungsbeispiels eines bei der vorliegenden Erfindung verwendeten Sensorelements; und
- Fig. 3: eine schematische Darstellung zur Veranschaulichung des Effekts einer Belagbildung, der bei der vorliegenden Erfindung ausgenutzt wird.

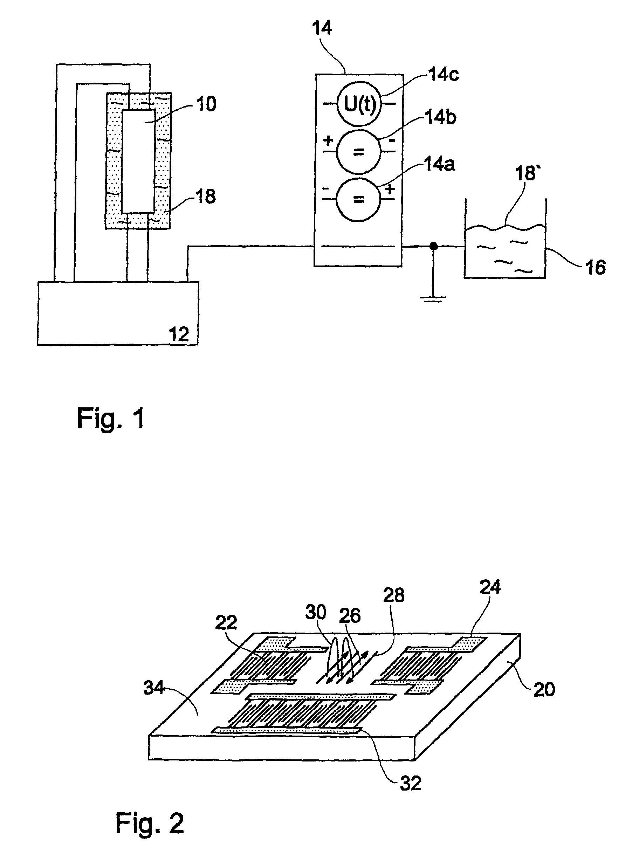

In Fig. 1 ist schematisch ein Sensorelement 10 gezeigt, das ein übliches Sensorelement zum Erfassen von physikalischen Parametern eines Schmiermittels, insbesondere eines Schmieröls, sein kann, beispielsweise ein Oberflächenwellensensor, wie er in den Druckschriften WO98/05953 und WO98/37412 beschrieben ist. Jedoch kann der Sensor ein beliebiger Sensor zur Erfassung physikalischer Parameter sein, dessen Ausgangssignal von einer an demselben, bzw. einer Sensoroberfläche desselben, anliegenden Flüssigkeit, insbesondere eines Schmieröls, abhängt. Ein bevorzugtes Ausführungsbeispiel eines solchen Sensors wird später bezugnehmend auf Fig. 2 näher erläutert. Beispielsweise kommt hier auch ein Schwingquarz als Sensorelement zur Erfassung der Viskosität in Betracht.

Der Sensor 10 ist mit einer Sensorelektronik 12 verbunden, die Auswertungsschaltungen zum Auswerten der von dem Sensorelement 10 erzeugten Ausgangssignale enthält. Die Sensorelektronik 12, die neben den Auswerteschaltungen ferner die Steuerung der erfindungsgemäßen Vorrichtung enthält, ist überdies mit einer Spannungsquelle 14 verbunden, die das Anlegen einer definierten Potentialdifferenz zwischen einer Ölwanne 16 und dem Sensorelement 10 ermöglicht. An dieser Stelle sei angemerkt, daß die Ölwanne 16 hier lediglich beispielhaft genannt ist, wobei die Potentialdifferenz zwischen Schmieröl und Sensorelement auch an anderer Stelle angelegt werden kann, beispielsweise zwischen anderen Teilen des Motorblocks und dem Sensorelement.

Wie in Fig. 1 dargestellt ist, kann die Ölwanne 16 als auf Masse liegend betrachtet werden. In Fig. 1 ist schematisch ferner ein Teil des Schmiermittels 18 gezeigt, das in Berührung mit dem Sensorelement 10 ist, insbesondere mit einer Erfassungsoberfläche, d.h. Sensoroberfläche, desselben. Die Spannungsquelle 14 ist vorzugsweise eine steuerbare Spannungsquelle, die das Anlegen fester Potentialdifferenzen vorgegebener Polaritäten 14a, 14b sowie das Anlegen zeitlich variierender Potentialdifferenzen 14c zwischen Schmiermittel und Sensorelement ermöglicht.

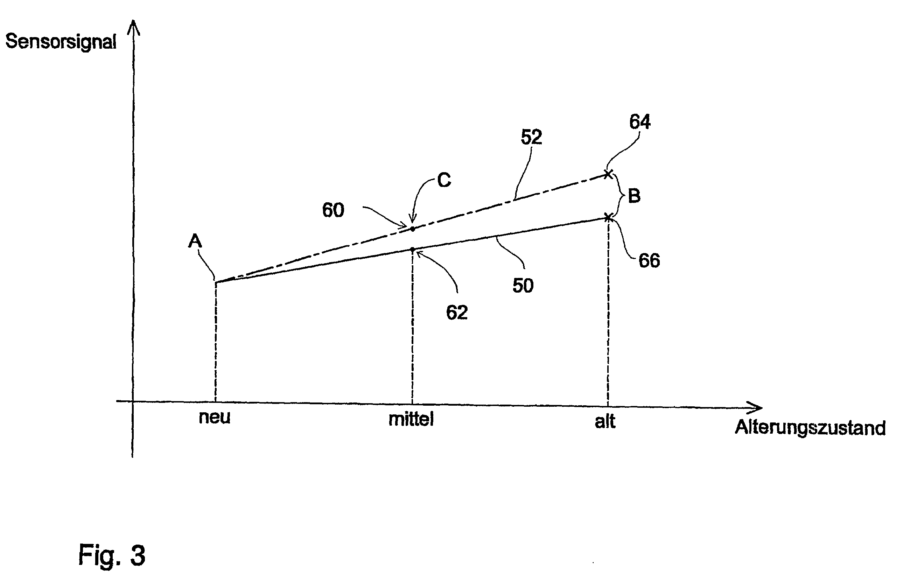

Hinsichtlich Fig. 1 ist anzumerken, daß das dargestellte Schmiermittel 18 in fluidischer Verbindung mit dem in der Ölwanne 16 angeordneten Schmiermittel 18' ist, so daß das Anlegen einer Spannung zwischen der Ölwanne 16 und dem Sensorelement 10 das Vorliegen einer Potentialdifferenz zwischen Schmiermittel 18 und Sensorelement 10 bewirkt. Somit ermöglicht die in Fig. 1 dargestellte Vorrichtung das Anlegen unterschiedlicher Potentialdifferenzen zwischen Sensorelement 10 und Schmiermittel 18, um basierend auf den bei zumindest zwei unterschiedlichen Potentialzuständen erhaltenen Ausgangssignalen des Sensorelements 10 Rückschlüsse auf eine Belagbildung auf dem Sensorelement 10 durch das Schmiermittel 18 zu ermöglichen, wie später bezugnehmend auf Fig. 3 näher erläutert wird.

Zunächst wird jedoch ein bevorzugtes Ausführungsbeispiel eines Sensorelements anhand Fig. 2 näher erläutert. Das dort dargestellte Sensorelement umfaßt ein Quarzsubstrat 20, auf dem zwei Interdigitalwandler 22, 24 angeordnet sind, um in einem sensitiven Bereich 26 eine Oberflächenwelle, die durch Pfeile 28 angezeigt ist, zu erzeugen. Ferner ist in Fig. 2 schematisch ein elektrisches Feld 30 gezeigt. Auf dem Quarzsubstrat 20 ist überdies ein Interdigitalkondensator 32 angeordnet. Ein solches Sensorelement, wie es in Fig. 2 gezeigt ist, kann basierend auf den Grundsätzen aufgebaut sein, die in der WO 98/05953 und der WO 98/37412 beschrieben sind. Dabei dienen die Interdigitalwandler 22, 24 zur Erfassung der Viskosität einer an der Sensoroberfläche 34 vorliegenden Flüssigkeit, wobei die Dämpfung bzw. Frequenz der angeregten Oberflächenwelle 28 von der angrenzenden Flüssigkeit, insbesondere der Viskosität derselben, abhängen. So nimmt die Dämpfung der angeregten Oberflächenwelle 28 mit zunehmender Viskosität des angrenzenden Schmieröls zu, während die Frequenz der angeregten Oberflächenwelle mit zunehmender Viskosität des angrenzenden Schmiermittels abnimmt. Ferner ist anzumerken, daß die Viskosität des Schmiermittels mit zunehmender Alterung desselben in der Regel zunimmt.

Der Interdigitalkondensator 32 dient zur Erfassung der Dielektrizitätskonstante bzw. der Leitfähigkeit des angrenzenden Schmiermittels, wobei die Leitfähigkeit und die Dielektrizitätszahl mit zunehmendem Alterungszustand des Schmiermittels in der Regel zunehmen. Neben dem beschriebenen Interdigitalkondensator können erfindungsgemäß auch andere kapazitive Sensoren verwendet werden.

Abweichend von dem in Fig. 2 dargestellten Sensorelement kann auch ein Sensorelement verwendet werden, bei dem einer der Interdigitalwandler des Oberflächenwellensensors gleichzeitig als Interdigitalkondensator verwendet wird, wobei diesbezüglich ebenfalls auf die Offenbarung der WO 98/37412 verwiesen sei. Ferner ist anzumerken, daß überdies mittels geeigneter Sensoreinrichtungen Temperatur und Druck des Schmiermittels erfaßt werden können, um die jeweiligen Abhängigkeiten des oder der erfaßten physikalische Parameter des Schmiermittels, beispielsweise der Viskosität, der Dielektrizitätszahl und der Leitfähigkeit von Temperatur und Druck bei der Beurteilung des Alterungszustands des Schmiermittels berücksichtigen zu können. Neben dem bezugnehmend auf Fig. 2 beschriebenen Sensorelement, das zur Erfassung von Viskosität, Dielektrizitätszahl und Leitfähigkeit dient, können andere Sensoren, die auf Änderungen der physikalischen Randbedingungen an der Grenzfläche Sensor-Schmiermittel reagieren, verwendet werden.

Anhand Fig. 3 wird nun nachfolgend erläutert, wie erfindungsgemäß beurteilt werden kann, ob ein Schmiermittel eine Belagbildung auf einem Sensorelement bewirkt. Insbesondere wird dies anhand des Beispiels der Erfassung der Viskosität eines Schmiermittels unter Verwendung der bezugnehmend auf die Fig. 1 und 2 beschriebenen Vorrichtung erläutert.

In Fig. 3 ist auf der Ordinate das die Viskosität anzeigende Sensorsignal aufgetragen, während auf der Abszisse der Alterungszustand, rein schematisch unterteilt in neu, mittel und alt, aufgetragen ist. An dieser Stelle sei angemerkt, daß die Darstellung in Fig. 3 rein schematisch ist, wobei der dargestellte Verlauf der Viskosität in keiner Weise an einen natürlichen Verlauf derselben angepaßt ist. Ferner sei angemerkt, daß die nachfolgenden Erläuterungen statt anhand der Viskosität auch anhand eines anderen physikalischen Parameters stattfinden könnten, wobei anzumerken ist, daß zur Auswertung auch ein für einen physikalischen Parameter des zu untersuchenden Schmiermittels spezifischer Parameter, beispielsweise die Frequenz einer Oberflächenwelle, die spezifisch für die Viskosität des Schmiermittels ist, verwendet werden kann. Dabei ist zu beachten, daß die Frequenz der Oberflächenwelle mit zunehmendem Alterungszustand des Schmiermittels abnimmt.

In Fig. 3 ist nun durch eine Kurve 50 der Verlauf der Viskosität bei einem zunehmenden Alterungszustand des Schmiermittels, das an der Sensoroberfläche 34 (Fig. 2) vorliegt, gezeigt. Wie zu erkennen ist, nimmt die Viskosität mit zunehmendem Alterungszustand von "neu" über "mittel" nach "alt" zu. Die Kurve 50 stellt eine über die Frequenz bzw. die Dämpfung einer Oberflächenwelle erfaßte Viskosität ohne das Vorliegen eines Belags auf dem Sensorelement dar. Dagegen stellt die strichpunktierte Kurve 52 den Viskositätsverlauf bei Vorliegen eines Belags auf der Sensoroberfläche dar, der durch das Vorliegen einer Potentialdifferenz einer ersten Polarität zwischen Sensorelement und Schmiermittel bewirkt wird. Wie aus Fig. 3 zu erkennen ist, findet bei einem neuwertigen Schmiermittel keine Belagbildung statt, Punkt A, während bei einem gealterten Schmiermittel eine Belagbildung B stattfindet, die sich durch eine Zunahme der Viskosität äußert. An dieser Stelle sei nochmals darauf hingewiesen, daß der Verlauf der Kurven 50 und 52 rein schematischer Natur ist.

Diese Belagbildung, die durch gealtertes Schmiermittel bewirkt wird, kann nun durch die Auswerteeinrichtung in der Sensorelektronik 12 erfaßt werden. Zu diesem Zweck werden zwei Werte des physikalischen Parameters, im vorliegenden Fall der Viskosität, bei unterschiedlichem Potentialzuständen zwischen Sensorelement und Schmiermittel erfaßt. Als Potentialzustände kommen dabei in Betracht: keine Potentialdifferenz zwischen Sensorelement und Schmiermittel angelegt, eine positive Potentialdifferenz zwischen Sensorelement und Schmiermittel angelegt (14b in Fig. 1) und eine negative Potentialdifferenz zwischen Sensorelement und Schmiermittel angelegt (14a in Fig. 1). Ergeben sich beim Vorliegen unterschiedlicher Potentialzustände zwischen Sensorelement und Schmiermittel verschiedene Ausgangssignale des Sensorelements, so ist dies eine Anzeige dafür, daß ein Schmiermittel mit der Sensoroberfläche in Berührung ist, das eine Belagbildung bewirkt, d.h. daß das Schmiermittel einen fortgeschrittenen Alterungszustand aufweist.

Somit kann durch Auswertung der zwei erfaßten Werte auf eine Belagbildung auf dem Sensorelement geschlossen werden, wobei auf der Grundlage dieser Belagbildung wiederum der Alterungszustand des Schmiermittels beurteilt werden kann.

Als bevorzugtes Ausführungsbeispiel der vorliegenden Erfindung sei im vorliegenden ein Fall erläutert, bei dem zum Erfassen eines ersten Werts des physikalischen Parameters eine erste Potentialdifferenz mit einer ersten Polarität und zum Erfassen des zweiten Werts eine zweite Potentialdifferenz mit zweiter Polarität zwischen Sensorelement und Schmiermittel erzeugt wird. Die zweite Polarität ist dabei entgegengesetzt zur ersten Polarität. Ferner sei angenommen, daß die erste Polarität eine Belagbildung auf der Sensoroberfläche bewirkt, wie sie in Fig. 3 dargestellt ist.

Werden nun die zwei Werte erfaßt, während ein neuwertiges Schmiermittel in Berührung mit der Sensoroberfläche vorliegt, befindet man sich jeweils in Punkt A, so daß keine Differenz zwischen den zwei Werten auftritt. Dies zeigt an, daß keine Belagbildung bewirkt wird, so daß auf ein neuwertiges Schmiermittel geschlossen werden kann.

Befindet sich hingegen ein Schmiermittel mittleren Alterungszustands in Berührung mit der Sensoroberfläche, befindet man sich im Bereich C (Fig. 3) und erhält bei Erfassung des ersten Werts einen Wert 60, während man bei Erfassung des zweiten Werts einen Wert 62 erfaßt. Dies ist darin begründet, daß durch das Anliegen der Potentialdifferenz mit der ersten Polarität zunächst ein Belag gebildet wird, der dann durch das Anliegen der Potentialdifferenz mit der zweiten Polarität wieder entfernt wird. Der Unterschied zwischen den Werten 60 und 62 zeigt an, daß eine Belagbildung auf dem Sensorelement stattfindet, wobei der Betrag der Differenz einen Rückschluß auf den bestimmten Alterungszustand des Schmiermittels zuläßt.

Liegt nun ein altes Schmiermittel in Berührung mit der Sensoroberfläche vor, ergibt sich in vergleichbarer Weise ein Unterschied B zwischen erstem erfaßten Wert 64 und zweitem erfaßten Wert 66. Somit kann hier auf eine stärkere Belagbildung und somit einen fortgeschritteneren Alterungszustand des Schmieröls geschlossen werden.

Es sei angemerkt, daß ein zu dem obigen Ergebnis vergleichbares Ergebnis auch erhalten wird, wenn die Polaritäten der angelegten Potentialdifferenzen umgekehrt sind, wobei dann im Fall eines Schmiermittels mit mittlerem Alterungszustand zunächst der Wert 62 und im Fall eines "alten" Schmiermittels zunächst der Wert 66 erfaßt werden würde.

Im obigen wurde davon ausgegangen, daß bei Erfassen des ersten Werts kein Belag auf der Sensoroberfläche vorlag. Das oben Gesagte gilt jedoch in analoger Weise auch dann, wenn beim Erfassen des ersten Werts ein Belag auf der Sensoroberfläche vorlag. Ein solcher Belag kann bei einem in einen Motor eingebauten Sensor durch eine inhärent zwischen Motoröl und Sensor vorliegende Potentialdifferenz, die nicht extern angelegt wird, erzeugt werden. Auch in diesem Fall wird bei vorliegen eines Potentialzustands einer Polarität ein Belag auf dem Sensorelement vorliegen, während beim Vorliegen eines Potentialzustands der anderen Polarität kein Belag vorliegt. Somit kann auch hier auf das Bewirken einer Belagbildung und somit dem Alterungszustand des Schmiermittels rückgeschlossen werden.

Bei dem oben beschriebenen Beispiel des Erfassens der Viskosität stellt jeweils der größere erfaßte Wert den Wert dar, der das Vorliegen eines Belags auf dem Sensorelement anzeigt. Somit kann aus diesem Wert die Polarität einer potentialdifferenz bestimmt werden, die eine Belagbildung auf dem Sensorelement bewirkt. Durch das Anlegen einer Potentialdifferenz mit entgegengesetzter Polarität kann dann bewirkt werden, daß der Belag von dem Sensorelement beseitigt wird.

Nach dem Beseitigen des Belags durch das Anlegen der Potentialdifferenz mit der entgegengesetzten Polarität können dann physikalische Parameter, im vorliegenden Beispiel die Viskosität, des zu untersuchenden Schmiermittels mittels des Sensorelements erfaßt werden, wobei diese Erfassung nicht durch irgendwelche Beläge auf dem Sensorelement verfälscht ist. Somit ermöglicht die vorliegende Erfindung zum einen die Erfassung eines weiteren Summenparameters, nämlich der Belagbildung, und zum anderen eine exaktere Erfassung von physikalischen Parametern, die bereits mittels bekannter Sensoren erfaßt wurden, beispielsweise der Viskosität, der Dielektrizitätszahl, der Leitfähigkeit, und dergleichen.

Obwohl oben bezugnehmend auf Fig. 3 die Erfassung der Viskosität als physikalischer Parameter erläutert wurde, besitzt die Erläuterung allgemeine Gültigkeit auch für die Erfassung der Leitfähigkeit bzw. der Dielektrizitätszahl und dergleichen, wobei jeweils darauf abzustellen ist, ob durch eine Belagbildung der jeweils erfaßte physikalische Parameter erhöht oder verringert wird. Dabei ist anzumerken, daß auch die bei Vorliegen eines Belags erfaßte Leitfähigkeit und Dielektrizitätskonstante höher oder niedriger sind als bei Fehlen eines Belags.

Es sei angemerkt, daß es auf die Höhe des angelegten Potentials erfindungsgemäß nicht ankommt, wobei es für einen Fachmann jedoch offensichtlich ist, daß das Anlegen einer Potentialdifferenz eines ausreichenden Betrags, um die in dem Schmiermittel befindlichen Partikel zu dem Sensorelement anzuziehen, anzulegen ist. In gleicher Weise ist es offensichtlich, daß die jeweilige Potentialdifferenz für einen geeigneten Zeitraum, beispielsweise einige Minuten, anzulegen ist, um das Aufbauen bzw. Abbauen des Belags zu ermöglichen.

Die Zeitdauer, für die eine Potentialdifferenz angelegt werden muß, um einen vollständigen Belagabbau zu bewirken, kann beispielsweise erfaßt werden, indem die Ausgangssignale des Sensorelements für einen vorbestimmten Zeitraum überwacht werden, während die Potentialdifferenz, die einen Abbau des Belags bewirkt, anliegt. Ab einem bestimmten Zeitpunkt wird eine Sättigung erreicht werden, d.h. das Ausgangssignal des Sensors gibt ausschließlich den Wert des physikalischen Parameters wieder, ohne daß ein Belag vorliegt. Die Zeitdauer bis zu diesem Sättigungswert kann dann als geeignete Zeitdauer verwendet werden. Anzumerken ist hierbei, daß diese Zeitdauer beispielsweise verringert werden kann, wenn die angelegte Potentialdifferenz erhöht wird.

Die vorliegende Erfindung eignet sich somit, um festzustellen, ob durch ein Schmiermittel eine Belagbildung auf einem Sensorelement bewirkt wird. Diese Feststellung kann dann verwendet werden, um den Alterungszustand des Öls zu bestimmen. Ferner kann diese Bestimmung verwendet werden, um eine Beseitigung eines Belags von dem Sensorelement sicherzustellen. Der belagfreie Sensor kann dann verwendet werden, um mittels des Sensorelements einen oder mehrere physikalische Parameter des Schmieröls exakt zu erfassen.

Die vorliegende Erfindung ermöglicht es ferner, die Alterung eines Schmieröls über einen Zeitraum hinweg, beispielsweise mehrere Monate bzw. Jahre, zu beobachten, indem der oder die physikalischen Parameter jeweils mit auf dem Sensorelement erzeugtem Belag und ohne denselben erfaßt werden. Die Erfassung einer solchen zeitlichen Entwicklung ermöglicht zuverlässige Aussagen über den Alterungszustand des Schmiermittels, insbesondere dann, wenn an sich gleiche Schmiermittel im neuwertigen Zustand Abweichungen der physikalischen Parameter aufweisen. Derartige Abweichungen können kompensiert werden, indem jeweils erfaßte Ist-Werte des physikalischen Parameters mit Soll-Werten desselben verglichen werden, die zu Beginn der Verwendung des Schmiermittels, beispielsweise nach einem Ölwechsel, als das neuwertige Schmiermittel beschreibend erfaßt werden.

Erfindungsgemäß werden vorzugsweise zwei Potentialzustände verwendet, die Potentialdifferenzen mit unterschiedlichem Vorzeichen definieren. Jedoch ist es auch möglich, daß ein Potentialzustand keine Potentialdifferenz zwischen Schmiermittel und Sensorelement definiert. Daneben können die Potentialzustände auch Potentialdifferenzen des gleichen Vorzeichens und verschiedener Höhe definieren, wobei dann als weiterer Faktor zur Beurteilung einer Belagbildung der zeitliche Verlauf des Aufbaus einer Belagbildung herangezogen werden kann.

Die vorliegende Erfindung schafft somit eine Vorrichtung und ein Verfahren, die auf der Grundlage der Erfassung, ob eine Belagbildung auf einem Sensorelement durch ein Schmiermittel bewirkt wird, eine zuverlässige Ermittlung des Alterungszustandes von Schmiermitteln, insbesondere Ölen, ermöglichen, wobei auf der Grundlage der Ermittlung des Alterungszustands dann ein Ölwechsel zum richtigen Zeitpunkt durchgeführt werden kann. Somit kann durch die vorliegende Erfindung zum einen eine Beschädigung des Motors durch zu langes Verwenden eines alten Öls verhindert werden, und zum anderen kann verhindert werden, daß zu früh ein Ölwechsel durchgeführt wird, so daß einer unnötigen Umweltverschmutzung entgegengewirkt werden kann.

## Patentansprüche

1. Vorrichtung zur Bestimmung, ob ein zu untersuchendes Schmiermittel (18) eine Belagbildung auf einem Sensorelement (10) bewirkt, mit folgenden Merkmalen:
einem Sensorelement (10) zum Erfassen von Werten zumindest eines physikalischen Parameters des Schmiermittels (18), das in Berührung mit dem Schmiermittel (18) angeordnet ist;
einer Einrichtung (14) zum Anlegen einer Potentialdifferenz zwischen Sensorelement (10) und Schmiermittel (18);
einer Einrichtung zur Auswertung von zumindest zwei Werten des physikalischen Parameters, die bei unterschiedlichen Potentialzuständen zwischen Sensorelement (10) und Schmiermittel (18) erfaßt werden, um zu bestimmen, ob eine Belagbildung auf dem Sensorelement (10) durch das Schmiermittel (18) bewirkt wird.

2. Vorrichtung nach Anspruch 1, die eine Einrichtung zum Ermitteln des Alterungszustands des Schmiermittels (18) abhängig von der Bestimmung, ob eine Belagbildung bewirkt wird, aufweist.

3. Vorrichtung nach Anspruch 1, die eine Einrichtung zur Bestimmung des Vorzeichens der Polarität einer Potentialdifferenz zwischen Sensorelement (10) und Schmiermittel (18), die eine Belagbildung durch das Schmiermittel (18) auf dem Sensorelement (10) bewirkt, und eine Einrichtung zum Beseitigen eines Belags auf dem Sensorelement (10) durch Anlegen einer Potentialdifferenz einer Polarität mit einem zu dem bestimmten Vorzeichen umgekehrten Vorzeichen zwischen Sensorelement (10) und Schmiermittel (18) aufweist.

4. Vorrichtung nach Anspruch 3, die ferner eine Einrichtung zur Ermittlung des Alterungszustands des Schmiermittels (18) basierend auf einem Wert des physikalischen Parameters, der erfaßt wird, nachdem ein Belag von dem Sensorelement (10) beseitigt wurde, aufweist.

5. Vorrichtung nach einem der Ansprüche 1 bis 4, bei der das Sensorelement (10) einen Oberflächenwellensensor (22, 24) oder einen Schwingquarz zum Erfassen der Viskosität des Schmiermittels (18) und/oder einen kapazitiven Sensor (32) zum Erfassen der Dielektrizitätszahl und/oder der Leitfähigkeit des Schmiermittels umfaßt.

6. Vorrichtung nach einem der vorhergehenden Ansprüche, bei der die Einrichtung zum Anlegen einer Potentialdifferenz eine Spannungsquelle (14) ist, die ausgelegt ist, um die Potentialdifferenz zwischen einem Motorblock, in dem das Schmiermittel (18) angeordnet ist, und dem Sensorelement (10) anzulegen.

7. Verfahren zum Bestimmen, ob ein zu untersuchendes Schmiermittel (18) eine Belagbildung auf einem Sensorelement (10) zum Erfassen von Werten zumindest eines physikalischen Parameters des Schmiermittels (18), das in Berührung mit dem Schmiermittel (18) angeordnet ist, bewirkt:
Erzeugen unterschiedlicher Potentialzustände zwischen Sensorelement (10) und Schmiermittel (28) und Erfassen des Werts des physikalischen Parameters bei zumindest zwei unterschiedlichen Potentialzuständen;
Bestimmen, ob eine Belagbildung auf dem Sensorelement (10) durch das Schmiermittel (18) bewirkt wird, basierend auf den zumindest zwei erfaßten Werten des physikalischen Parameters.

8. Verfahren nach Anspruch 7, das ferner den Schritt des Ermitteins des Alterungszustands des Schmiermittels (18) basierend auf der Bestimmung, ob eine Belagbildung bewirkt wird, aufweist.

9. Verfahren nach Anspruch 7, das ferner folgende Schritte aufweist:
Bestimmen des Vorzeichens der Polarität einer Potentialdifferenz zwischen Sensorelement (10) und Schmiermittel (18), die eine Belagbildung durch das Schmiermittel (18) auf dem Sensorelement (10) bewirkt, und
Beseitigen eines Belags auf dem Sensorelement (10) durch Anlegen einer Potentialdifferenz einer Polarität mit einem zu dem bestimmten Vorzeichen umgekehrten Vorzeichen zwischen Sensorelement (10) und Schmiermittel (18).

10. Verfahren nach Anspruch 9, das ferner den Schritt des Ermittelns des Alterungszustands des Schmiermittels (18) basierend auf einem Wert des physikalischen Parameters, der erfaßt wird, nachdem ein Belag von dem Sensorelement (10) beseitigt wurde, aufweist.

11. Verfahren nach einem der Ansprüche 7 bis 10, bei dem als Sensorelement (10) ein Oberflächenwellensensor (22, 24) oder ein Schwingquarz zum Erfassen der Viskosität des Schmiermittels (18) und/oder ein kapazitiver Sensor (32) zum Erfassen der Dielektrizitätszahl und/oder der Leitfähigkeit des Schmiermittels verwendet wird.

12. Verfahren nach einem der Ansprüche 7 bis 11, bei dem die unterschiedlichen Potentialzustände durch Anlegen einer Spannung zwischen einem Motorblock, in dem das Schmiermittel (18) angeordnet ist, und dem Sensorelement (10) erzeugt werden.

## Claims

1. A device for determining whether a lubricant (18) to be examined causes the formation of a deposit on a sensor element (10), comprising:
a sensor element (10) for detecting values of at least one physical parameter of the lubricant (18), said sensor element (10) being arranged in contact with the lubricant (18);
a means (14) for applying a potential difference between the sensor element (10) and the lubricant (18);
a means for evaluating at least two values of the physical parameter that are detected at different potential states between the sensor element (10) and the lubricant (18), in order to thus determine whether the lubricant (18) causes a deposit formation on the sensor element (10).

2. A device according to claim 1 comprising a means for determining the aging condition of the lubricant (18) responsive to said determination as to whether a deposit formation is caused.

3. A device according to claim 1 comprising a means for determining the sign of the polarity of a potential difference between the sensor element (10) and the lubricant (18) which causes a deposit formation by the lubricant (18) on the sensor element (10), and a means for eliminating a deposit on the sensor element (10) by application of a potential difference of a polarity of opposite sign to said determined sign between said sensor element (10) and said lubricant (18).

4. A device according to claim 3, further comprising a means for determining the aging condition of the lubricant (18) on the basis of a value of the physical parameter that is detected after elimination of a deposit from the sensor element (10).

5. A device according to any of claims 1 to 4, wherein the sensor element (10) comprises a surface-wave sensor (22, 24) or a quartz oscillator for detecting the viscosity of the lubricant (18) and/or a capacitive sensor (32) for detecting the relative permittivity and/or the conductivity of the lubricant.

6. A device according to any of the preceding claims, wherein said means for applying a potential difference is a voltage source (14) arranged to apply the potential difference between an engine block, in which the lubricant (18) is arranged, and said sensor element (10).

7. A method of determining whether a lubricant (18) to be examined effects the formation of a deposit on a sensor element (10) for detecting values of at least one physical parameter of the lubricant (18), said sensor element (10) being arranged in contact with the lubricant (18), comprising the steps of:
generating different potential states between the sensor element (10) and the lubricant (18) and detecting the value of the physical parameter in at least two different potential states;
determining whether the lubricant (18) causes the formation of a deposit on the sensor element (10), on the basis of the at least two detected values of the physical parameter.

8. A method according to claim 7, further comprising the step of determining the aging condition of the lubricant (18) on the basis of the determination as to whether a deposit formation is caused.

9. A method according to claim 7, further comprising the steps of:
determining the sign of the polarity of a potential difference between the sensor element (10) and the lubricant (18) which causes a deposit formation by the lubricant (18) on the sensor element (10), and
eliminating a deposit on the sensor element (10) by application of a potential difference of a polarity of opposite sign to said determined sign between said sensor element (10) and said lubricant (18).

10. A method according to claim 9, further comprising the step of determining the aging condition of the lubricant (18) on the basis of a value of the physical parameter that is detected after elimination of a deposit from the sensor element (10).

11. A method according to any of claims 7 to 10, wherein the sensor element (10) used is a surface-wave sensor (22, 24) or a quartz oscillator for detecting the viscosity of the lubricant (18) and/or a capacitive sensor (32) for detecting the relative permittivity and/or the conductivity of the lubricant.

12. A method according to any of claims 7 to 11, wherein said different potential states are produced by application of a voltage between an engine block in which the lubricant is arranged, and said sensor element (10).

## Revendications

1. Dispositif pour détecter si un lubrifiant à examiner (18) entraîne la formation d'un dépôt sur un élément capteur (10), aux caractéristiques suivantes :
un élément capteur (10) destiné à capter des valeurs d'au moins un paramètre physique du lubrifiant (18), disposé en contact avec le lubrifiant (18) ;
un dispositif (14) destiné à appliquer une différence de potentiel entre l'élément capteur (10) et le lubrifiant (18) ;
un dispositif destiné à évaluer au moins deux valeurs du paramètre physique captées à différents états de potentiel entre l'élément capteur (10) et le lubrifiant (18), pour déterminer si une formation de dépôt est entraînée sur l'élément capteur (10) par le lubrifiant (18).

2. Dispositif selon la revendication 1, présentant un dispositif destiné à déterminer l'état de vieillissement du lubrifiant (18) en fonction de la détermination de s'il est entraîné une formation de dépôt.

3. Dispositif selon la revendication 1, présentant un dispositif destiné à déterminer le signe de la polarité d'une différence de potentiel entre l'élément capteur (10) et le lubrifiant (18) entraînant une formation de dépôt par le lubrifiant (18) sur l'élément capteur (10) et un dispositif destiné à éliminer un dépôt sur l'élément capteur (10) par l'application d'une différence de potentiel d'une polarité à signe contraire au signe déterminé entre l'élément capteur (10) et le lubrifiant (18).

4. Dispositif selon la revendication 3, présentant, par ailleurs, un dispositif destiné à déterminer l'état de vieillissement du lubrifiant (18) sur base d'une valeur du paramètre physique qui est capté après qu'un dépôt a été éliminé de l'élément capteur (10).

5. Dispositif selon l'une des revendications 1 à 4, dans lequel l'élément capteur (10) comporte un capteur d'ondes de surface (22, 24) ou un quartz oscillant destiné à capter la viscosité du lubrifiant (18) et/ou un capteur capacitif (32) destiné à capter le nombre diélectrique et/ou la conductivité du lubrifiant.

6. Dispositif selon l'une des revendications précédentes, dans lequel le dispositif destiné à appliquer une différence de potentiel est une source de tension (14) conçue pour appliquer la différence de potentiel entre un bloc moteur dans lequel est disposé le lubrifiant (18) et l'élément capteur (10).

7. Procédé pour détecter si un lubrifiant à examiner (18) entraîne la formation d'un dépôt sur un élément capteur (10) destiné à capter des valeurs d'au moins un paramètre physique du lubrifiant (18), disposé en contact avec le lubrifiant (18) :
générer différents états de potentiel entre l'élément capteur (10) et le lubrifiant (18) et capter la valeur du paramètre physique à au moins deux états de potentiel différents ;
déterminer si une formation de dépôt est entraînée sur l'élément capteur (10) par le lubrifiant (18), sur base des au moins deux valeurs captées du paramètre physique.

8. Procédé selon la revendication 7, présentant, par ailleurs, l'étape consistant à déterminer l'état de vieillissement du lubrifiant (18) sur base de la détermination de s'il est entraîné une formation de dépôt.

9. Procédé selon la revendication 7, présentant, par ailleurs, les étapes suivantes consistant à :
déterminer le signe de la polarité d'une différence de potentiel entre l'élément capteur (10) et le lubrifiant (18) entraînant une formation de dépôt par le lubrifiant (18) sur l'élément capteur (10), et
éliminer un dépôt sur l'élément capteur (10) par l'application d'une différence de potentiel d'une polarité à signe contraire au signe déterminé entre l'élément capteur (10) et le lubrifiant (18).

10. Procédé selon la revendication 9, comprenant, par ailleurs, l'étape consistant à déterminer l'état de vieillissement du lubrifiant (18) sur base d'une valeur du paramètre physique qui est captée après qu'un dépôt a été éliminé de l'élément capteur (10).

11. Procédé selon l'une des revendications 7 à 10, dans lequel il est utilisé, comme élément capteur (10), un capteur d'ondes de surface (22, 24) ou un quartz oscillant destiné à capter la viscosité du lubrifiant (18) et/ou un capteur capacitif (32) destiné à capter le nombre diélectrique et/ou la conductivité du lubrifiant.

12. Procédé selon l'une des revendications 7 à 11, dans lequel les différents états de potentiel sont générés par l'application d'une tension entre un bloc moteur dans lequel est disposé le lubrifiant (18) et l'élément capteur (10).
